(19) Europäisches Patentamt
European Patent Office
Office européen des brevets

(11) Veröffentlichungsnummer: **0 120 254**
**A1**

(12) # EUROPÄISCHE PATENTANMELDUNG

(21) Anmeldenummer: **84101435.0**

(22) Anmeldetag: **13.02.84**

(51) Int. Cl.$^3$: **C 08 G 71/04**
C 08 G 18/72, C 08 G 18/83
C 07 C 125/06, C 07 C 118/02
C 08 G 18/30

(30) Priorität: **26.02.83 DE 3306845**

(43) Veröffentlichungstag der Anmeldung:
**03.10.84 Patentblatt 84/40**

(84) Benannte Vertragsstaaten:
**BE DE FR GB IT**

(71) Anmelder: **BAYER AG**
**Konzernverwaltung RP Patentabteilung**
**D-5090 Leverkusen 1 Bayerwerk(DE)**

(72) Erfinder: **Rasshofer, Werner, Dr.**
**Wolfskaul 10**
**D-5000 Köln 80(DE)**

(54) **Verfahren zur Herstellung von Polyisocyanaten, die nach diesem Verfahren erhältlichen Polyisocyanate, sowie ihre Verwendung zur Herstellung von Polyurethanen oder von Polyaminen.**

(57) Ein neues Verfahren zur Herstellung von Polyisocyanaten durch 1- oder 2-stufige Phosgenierung von primären Polyaminen bzw. ihrer phosgenierbarer Salze oder Kohlendioxid-Addukte, wobei die Phosgenierung zumindest in der zweiten Stufe in Gegenwart von primären und/oder sekundären Alkoholen unter Einhaltung eines Äquivalentverhältnisses von Aminogruppen zu Hydroxylgruppen von 1:0,005 bis 1:0,99 erfolgt, die nach dem Verfahren erhältlichen Polyisocyanate, sowie ihre Verwendung als Ausgangsmaterialien bei der Herstellung von Polyurethankunststoffen bzw. zur Herstellung der ihnen entsprechenden Polyamine durch hydrolytische Überführung ihrer Isocyanatgruppen in Aminogruppen.

EP 0 120 254 A1

BAYER AKTIENGESELLSCHAFT 5090 Leverkusen, Bayerwerk

Konzernverwaltung RP
Patentabteilung Wr/by-c

# Verfahren zur Herstellung von Polyisocyanaten, die nach diesem Verfahren erhältlichen Polyisocyanate, sowie ihre Verwendung zur Herstellung von Polyurethanen oder von Polyaminen

Die vorliegende Erfindung betrifft ein neuartiges Verfahren zur Herstellung von Polyisocyanaten durch Phosgenierung der ihnen zugrundeliegenden Polyamine bzw. ihrer phosgenierbaren Salze oder Kohlendioxid-Addukte unter Mitverwendung von, alkoholische Hydroxylgruppen aufweisenden, Verbindungen, die nach diesem Verfahren erhältlichen Verbindungen und ihre Verwendung als Aufbaukomponente bei der Herstellung von Polyurethankunststoffen bzw. als Ausgangsmaterialien zur Herstellung der ihnen entsprechenden Polyamine.

Bei der Herstellung von Polyurethankunststoffen aus Polyisocyanaten und Polyhydroxylverbindungen stellt das sogenannte Prepolymer- bzw. Semiprepolymer-Verfahren eine wichtige Variante dar. Bei diesem Verfahren wird zunächst aus überschüssigem Polyisocyanat und der Polyhydroxylkomponente ein, freie Isocyanatgruppen

aufweisendes, Vorpolymeres (Prepolymer) oder ein Gemisch aus derartigem Vorpolymer mit überschüssigem monomerem Ausgangspolyisocyanat (Semiprepolymer) hergestellt, welche Vorprodukte in einem zweiten Reaktionsschritt mit einem Kettenverlängerungsmittel zum hochmolekularen Polyurethan umgesetzt werden (vgl. z.B. High Polymers, Vol. XVI "Polyurethanes", Part I von J.H. Saunders und K.C. Frisch, Interscience Publishers (1962) oder Kunststoff-Handbuch, Band VII, "Polyurethane" von R.Vieweg und A. Höchtlen, Carl Hanser Verlag München (1966)).

Nachteilig bei diesem Verfahren ist, daß zur Herstellung der NCO-Prepolymeren bzw. NCO-Semiprepolymeren ein gesonderter Reaktionsschritt durchgeführt werden muß, bei dem ein Teil der NCO-Gruppen des Ausgangspolyisocyanats mit den alkoholischen Hydroxylgruppen der Hydroxylkomponente zur Reaktion gebracht wird.

Es war daher die der Erfindung zugrundeliegende Aufgabe, der Fachwelt ein einfacheres Verfahren zur Herstellung von NCO-Prepolymeren bzw. von NCO-Semiprepolymeren zur Verfügung zu stellen. Weiterhin war es eine der vorliegenden Erfindung zugrundeliegende Aufgabe, ein Verfahren zur Verfügung zu stellen, bei dem der bei der großtechnischen Herstellung von destillierbaren Polyisocyanaten wie z.B. Toluylendiisocyanat oder Hexamethylendiisocyanat anfallende Destillationsrückstand in einer für weitere Umsetzungen verwertbaren, flüssigen Konsistenz anfällt.

Wie jetzt überraschend gefunden wurde, gelingt es, diese Aufgaben durch das nachstehend näher beschriebene erfindungsgemäße Verfahren zu lösen, bei welchem bei der Phosgenierung von primären Polyaminen die in den klassischen NCO-Prepolymeren bzw. NCO-Semiprepolymeren über Urethangruppen eingebauten Polyhydroxylverbindungen zusammen mit dem primären Polyamin der Phosgenierung unterzogen werden, so daß unmittelbar in einem Reaktionsschritt die entsprechenden NCO-Prepolymeren bzw. NCO-Semiprepolymeren anfallen. Darüber hinaus gestattet das erfindungsgemäße Verfahren bei der Phosgenierung von primären Polyaminen unter Mitverwendung von unterschüssigen Mengen an ein- und mehrwertigen Alkoholen die Herstellung von destillierbaren Polyisocyanaten, deren Destillationsrückstände, beispielsweise im Unterschied zu den Destillationsrückständen, wie sie bei der destillativen Aufarbeitung von Diisocyanato-toluolen anfallen, durch die erfindungsgemäße Modifizierung in einer besser fließfähigen und weiter verarbeitbaren Konsistenz anfallen.

Gegenstand der Erfindung ist ein Verfahren zur Herstellung von Polyisocyanaten durch ein- oder zweistufige Phosgenierung von primären Polyaminen, gegebenenfalls nach ihrer an sich bekannten Überführung in phosgenierbare Salze oder Kohlendioxid-Addukte, dadurch gekennzeichnet, daß man die Phosgenierung zumindest in der zweiten Stufe in Gegenwart von mindestens einer, mindestens eine primäre und/oder sekundäre alkoholische Hydroxylgruppe aufweisenden Verbindung unter Einhaltung eines Äquivalentverhältnisses von im Ausgangsamin vorliegenden, primären Aminogruppen zu Hydroxylgruppen von 1:0,005 bis 1:0,99 durchführt.

Gegenstand der Erfindung sind auch die nach diesem Verfahren erhältlichen Polyisocyanate.

Gegenstand der Erfindung ist auch die Verwendung der nach diesem Verfahren erhältlichen Polyisocyanate als Aufbaukomponente bei der Herstellung von Polyurethankunststoffen nach dem Isocyanat-Polyadditionsverfahren und als Ausgangsmaterial zur Herstellung der ihnen entsprechenden Polyamine durch hydrolytische Überführung ihrer Isocyanatgruppen in Aminogruppen.

Neben der Einsparung eines Verfahrensschritts weist das erfindungsgemäße Verfahren im Vergleich zu den bekannten zweistufigen Verfahren zur Herstellung von NCO-Prepolymeren noch eine Reihe weiterer Vorteile auf: Bei der Durchführung des erfindungsgemäßen Verfahrens muß nicht mit freien monomeren Polyisocyanaten gearbeitet werden, was entsprechende sicherheitstechnische Maßnahmen überflüssig macht. Die Herstellung von weitgehend monomerenfreien NCO-Prepolymeren gelingt beim erfindungsgemäßen Verfahren einfach durch geeignete Wahl des Äquivalentverhältnisses zwischen Aminogruppen und Hydroxylgruppen beispielsweise innerhalb des Bereichs von 1:0,5 bis 1:0,99, ohne daß eine nachträgliche Entfernung von nicht umgesetzten Ausgangsisocyanaten beispielsweise durch Dünnschichtdestillation erforderlich wäre. Im übrigen eignet sich das erfindungsgemäße Verfahren zur Herstellung von NCO-Prepolymeren auf Basis beliebiger Polyisocyanate, während man beim alten zweistufigen Verfahren oft darauf achten mußte, nur solche Diisocyanate einzusetzen, die sich durch Isocyanatgruppen einer unterschiedlichen Reaktivität auszeichnen.

Bei den beim erfindungsgemäßen Verfahren als Ausgangsmaterialien einzusetzenden Polyaminen handelt es sich um beliebige, mindestens zwei primäre Aminogruppen aufweisende und ansonsten unter den Reaktionsbedingungen inerte organische Verbindungen des Molekulargewichtsbereichs 60 bis 500, vorzugsweise 108 bis 250. Aromatische Polyamine eines innerhalb dieser Bereiche liegenden Molekulargewichts sind besonders bevorzugte Ausgangsmaterialien.

Beispiele geeigneter aromatischer Diamine sind 2,4-Diaminotoluol, 2,6-Diaminotoluol, 1,4-Diaminobenzol, 2,4-Diaminomesitylen, 1,3,5-Triethyl-2,4-diaminobenzol, 1,3,5-Triisopropyl-2,4-diaminobenzol, 1-Methyl-3,5-diethyl-2,4-diaminobenzol, 1-Methyl-3,5-diethyl-2,6-diaminobenzol, 4,6-Dimethyl-2-ethyl-1,3-diaminobenzol, 1,5-Diaminonaphthalin, 1,8-Diaminonaphthalin, 2,7-Diaminonaphthalin, 4,4'-Diaminodiphenylmethan, 2,4'-Diaminodiphenylmethan, 2,2'-Diaminodiphenylmethan, 3,3'-Dimethyl-4,4'-diaminodiphenylmethan, 4-Methyl-3,4'-diaminodiphenylmethan, 3,5,3',5'-Tetraethyl-4,4-diaminodiphenylmethan, 3,5,3',5'-Tetraisopropyl-4,4'-diaminodiphenylmethan, 3,5-Diethyl-3',5'-diisopropyl-4,4'-diaminodiphenylmethan, 2,2-Bis-(4-aminophenyl)-propan, 1,1-Bis-(4-aminophenyl)-cyclohexan, 1,1-Bis-(4-amino-3-methylphenyl)-cyclohexan, ferner 3,5- und 2,4-Diaminobenzoesäureester gemäß DE-OS 2 025 900, estergruppenhaltige Diamine gemäß DE-OS 1 803 635, 2 040 650 und 2 160 589, ethergruppenaufweisende Diamine gemäß DE-OS 1 770 525 und 1 809 172 (US-PS 3 654 364 und 3 736 295), gegebenenfalls in 5-Stellung substituierte 2-Halogen-1,3-phenylendiamine (DE-OS 2 001 772, 2 025 896 und 2 065 869), 3,3'-Dichlor-4,4'-diaminodiphenylmethan, 4,4'-Diaminodiphenylsulfide gemäß DE-OS 2 404 976, Diaminodiphenylthioether gemäß DE-OS 2 509 404, durch Alkylthiogruppen substituierte aromatische Diamine gemäß DE-OS 2 638 760, Diaminobenzolphosphorsäureester gemäß DE-OS 2 459 491, Sulfonat- oder Carboxylatgruppen enthaltende aromatische Diamine gemäß DE-OS 2 720 166 sowie die in der DE-OS 2 635 400 aufgeführte hochschmelzende Diamine, Aminoalkylthioaniline gemäß DE-OS 2 734 574 (aromatisch-aliphatische Diamine) und beliebige Gemische derartiger Polyamine.

Weniger bevorzugt sind aliphatische Polyamine wie z.B. lineare oder verzweigte aliphatische Diamine wie z.B. 1,2-Diaminoethan, 1,6-Diaminohexan, 2,2,4-Trimethyl-1,6-diaminohexan, 1,8-Diaminooctan, 1,5-Diamino-3-oxa-pentan, 1,8-Diamino-3,6-dioxaoctan, 1,11-Diamino-3,6,9-trioxaundecan, ferner cycloaliphatische Diamine wie 5-Amino-2,2,4-trimethyl-1-cyclopentanmethylamin, 5-Amino-2-aminomethyl-1,3,3-trimethylcyclohexan, 1,4-Diaminocyclohexan, 1,3-Diaminocyclohexan, 1,8-Diaminop-menthan, 1-Methyl-2,6-diaminocyclohexan, 1-Methyl-2,4-diaminocyclohexan, 4,4'-Diamino-dicyclohexylmethan sowie seine 2,4'- bzw. 2,2'-Isomeren, 4,4'-Diamino-3,3'-dimethyldicyclohexylmethan sowie seine 2,4'- und

2,2'-Isomeren, 4,4'-Diaminodicyclohexylethan, 4,4'-Diaminodicyclohexylether, Bis-(4-aminocyclohexyl)-propan-(2,2), 4,4'-Diaminodicyclohexan, 4,4'-Diamino-3,3'-diethyldicyclohexylmethan, 1,1-Di-(4'-aminocyclohexyl)-cyclohexan, 1,1-Di-(4'-amino-3'-methylcyclohexyl)-cyclohexan, 4,4'-Diamino-3,5-diethyl-3',5'-diisopropyldicyclohexylmethan, 4,4'-Diamino-3,3',5,5'-tetraethyldicyclohexylmethan bzw. deren Gemische.

Bei der Durchführung des erfindungsgemäßen Verfahrens werden die beispielhaft genannten Polyamine in Abmischung mit mindestens einer, mindestens eine primäre oder sekundäre alkoholische Hydroxylgruppe aufweisenden, ansonsten unter den Bedingungen des erfindungsgemäßen Verfahrens inerten organischen Verbindung des Molekulargewichtsbereichs 32 bis 10 000 eingesetzt. Versuchsweise weist diese alkoholische Komponente eine (mittlere) Hydroxylfunktionalität von 2 bis 3 und ein (mittleres) Molekulargewicht von 62 bis 6 000, insbesondere 400 bis 3 000 auf. Beispiele geeigneter Alkohole sind

a) Ether- und Estergruppen-freie Alkanpolyole, z.B. solche des Molekulargewichtsbereichs 62 - 250 wie z.B. Ethylenglykol, 1,2-Propandiol, 1,3-Propandiol, 1,2-, 1,3-, 2,3-, 1,4-Butandiol, 1,5-Pentandiol, Neopentylglykol, 1,6-Hexandiol, 2,5-Dimethyl-2,5-hexandiol, 2,2,4-Trimethyl-1,3-pentandiol, Trimethyl-1,6-hexandiole, 1,12-Octadecandiol, Trimethylolpropan, Glycerin, Trimethylolethan, Hexantriole, Sorbit, Mannit oder Pentaerythrit;

b) Ether- und Estergruppen-freie Cycloalkanpolyole, z.B. solche des Molekulargewichtsbereichs 116 - 250 wie z.B. 1,2-, 1,3- oder 1,4-Dihydroxycyclohexan, 2,2,4,4-Tetramethyl-1,3-cyclobutandiol, 1,4-Cyclohexan-dimethanol, 4,4'-Dihydroxy-dicyclohexylmethan oder 2,2-Bis-(4-hydroxycyclohexyl)-propan;

c) Ethergruppen aufweisende Polyole, z.B. solche des Molekulargewichtsbereichs 106 bis 10 000, wie z.B. Diethylenglykol, Triethylenglykol, Tetraethylenglykol, Dipropylenglykol, Tripropylenglykol, Tetrapropylenglykol oder die aus der Polyurethanchemie an sich bekannten Polyetherpolyole des obengenannten bevorzugten bzw. besonders bevorzugten Molekulargewichtsbereichs, wie sie in an sich bekannter Weise durch Alkoxylierung unter Verwendung von Ethylenoxid und/oder Propylenoxid von geeigneten Startermolekülen wie beispielsweise die unter a) und b) genannten Verbindungen erhalten werden;

d) Estergruppen aufweisende Polyole, z.B. solche des Molekulargewichtsbereichs 206 bis 10 000 wie z.B. Bernsteinsäure-bis-(2-hydroxyethyl)-ester, Adipinsäure-bis-(2-hydroxyethyl)-ester, und insbesondere die aus der Polyurethanchemie an sich bekannten höhermolekularen Polyesterpolyole des obengenannten bevorzugten bzw. besonders bevorzugten Molekulargewichtsbereichs, wie sie in an sich bekannter Weise durch Umsetzung von mehrwertigen Alkoholen der insbesondere oben unter a) genannten Art mit unterschüssigen Mengen an Dicarbonsäuren oder Dicarbonsäureanhydriden wie z.B. Adipinsäure, Phthalsäure, Phthalsäureanhydrid, Tetrahydrophthalsäure oder Tetrahydrophthalsäureanhydrid erhalten werden; und

e) beliebigen Gemischen der unter a) bis d) genannten Alkohole.

Die Alkoholkomponente enthält vorzugsweise ausschließlich mindestens zweiwertige Alkohole. Es ist jedoch grundsätzlich auch möglich, beim erfindungsgemäßen Verfahren einwertige Alkohole des Molekulargewichtsbereichs 32 bis ca. 250 wie z.B. Methanol, Ethanol, 1-Propanol, 2-Propanol, 1-Butanol, 1-Hexanol, 1-Decanol, 1-Octadecanol, 2-Methoxyethanol, Cyclohexanol, 2-Phenylethanol oder 1-Phenoxy-2-propanol bzw. Gemische derartiger einwertiger Alkohole als alleinige alkoholische Komponente einzusetzen bzw. in Kombination mit den mehrwertigen Alkoholen zu verwenden.

Andere geeignete, jedoch weniger bevorzugte Alkohole sind beispielsweise Heteroatome aufweisende Alkohole, wie beispielsweise Aminopolyetherpolyole, wie sie durch Alkoxylierung von Aminogruppen aufweisenden Verbindungen wie Ammoniak, Anilin, Phenylendiaminen, Ethanolamin oder Propanolamin erhalten werden; Polythioether, insbesondere die Kondensationsprodukte von Thiodiglykol mit sich

selbst und/oder mit anderen Glykolen, Dicarbonsäuren, Formaldehyd, Aminocarbonsäuren oder Aminoalkoholen; ferner geeignet sind Polyacetale wie z.B. die aus Glykolen wie Diethylenglykol oder Triethylenglykol und Formaldehyd herstellbaren Verbindungen oder Hydroxylgruppen aufweisende Polycarbonate wie z.B. solche, die durch Umsetzung von Diolen der beispielhaft genannten Art mit Diphenylcarbonat oder Phosgen hergestellt werden können.

Weitere Beispiele für erfindungsgemäß geeignete Alkohole für Hydroxylverbindungen sind z.B. in High Polymers, Vol. XVI, "Polyurethanes, Chemistry and Technology", verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München 1966, z.B. auf den Seiten 45-71, beschrieben.

Bei der Durchführung des erfindungsgemäßen Verfahrens kommen die Hydroxylgruppen aufweisenden Verbindungen in einer solchen Menge zum Einsatz, daß auf jede, im zu phosgenierenden Polyamin vorliegende primäre Aminogruppe 0,005 bis 0,99, vorzugsweise 0,1 bis 0,8 und insbesondere 0,1 bis 0,6 alkoholische Hydroxylgruppen entfallen.

Bei der Durchführung des erfindungsgemäßen Verfahrens wird in Analogie zu den bekannten Phosgenierverfahren primärer Amine vorgegangen, wie sie beispielsweise von

S. Petersen in Houben-Weyl/Müller, Handbuch der organischen Chemie, Thieme Verlag, 1952, Band VIII, Seiten 120 ff; von W. Siefken in Liebigs Annalen der Chemie, 562, 75 ff (1949), von J.H. Saunders und J.R. Slocombe in Chem. Rev. 43, 203 (1948) oder von A.A. Blagonravova und G.A. Levkovic in Uspechi Chim. 24, 93-119 (1955) beschrieben wurden. Bei der Durchführung der erfindungsgemäßen Phosgenierungsreaktion kann sowohl nach dem Einals auch nach dem Zweistufen-Verfahren (Kalt-/Heißphosgenierung) verfahren werden, wobei sowohl eine Direktphosgenierung der Polyamine als auch eine Phosgenierung ihrer phosgenierbaren Salze, insbesondere ihrer Hydrochloride als auch eine Phosgenierung ihrer Kohlendioxid-Addukte (Carbamat-Methode) in Betracht kommt. Die erfindungswesentliche Zugabe der alkoholischen Komponente kann hierbei sowohl zu Beginn (Verwendung von Gemischen aus Aminen und Alkoholen) als auch nach der gegebenenfalls durchgeführten Modifizierung der zu phosgenierenden Amine beispielsweise mittels Chlorwasserstoff oder Kohlendioxid oder im Falle der Zweistufen-Phosgenierung, auch im Anschluß an die Kaltphosgenierung, bei welcher die freien Aminogruppen in Hydrochlorid- und Carbamoylchlorid-Gruppen überführt werden, erfolgen. Bei der Phosgenierung aromatischer Ausgangsamine wird vorzugsweise nach dem Zweistufenverfahren (Kalt-/Heißphosgenierung der freien Amine), bei der Phosgenierung von aliphatischen oder cycloaliphatischen Polyaminen vorzugsweise nach der Carbamatmethode verfahren.

Die erfindungsgemäße Phosgenierungsreaktion wird im allgemeinen innerhalb des Temperaturbereichs von -20 bis +200°C unter Mitverwendung eines geeigneten inerten Lösungsmittels wie z.B. Chloroform, Dioxan, Dichlorethan, Phosphoroxichlorid und insbesondere Chlorbenzol bzw. Dichlorbenzol durchgeführt. Auf die Mitverwendung derartiger Hilfslösungsmittel kann u.U. dann verzichtet werden, wenn höhermolekulare Alkohole mit niedriger Hydroxylzahl mitverwendet werden, die die Funktion eines Lösungsmittels mit übernehmen können.

Grundsätzlich ist bei der Durchführung der erfindungsgemäßen Phosgenierungsreaktion darauf zu achten, daß die Reaktionsbedingungen so gewählt werden, daß eine Bildung von Carbonaten (Reaktion von Hydroxylgruppen mit Phosgen über die Zwischenstufe von Chlorkohlensäureestergruppen) und eine Bildung von Harnstoffen (Reaktion von Carbamoylgruppen mit freien Aminogruppen) weitgehend unterbleibt. Die erste der genannten Nebenreaktionen findet in nennenswertem Umfang erst bei vergleichsweise hohen Temperaturen statt. Zu ihrer Vermeidung ist es ausreichend, das Reaktionsgemisch aus (i) alkoholischer Komponente, (ii) gegebenenfalls in Form von phosgenierbaren Salzen, Carbamaten oder Hydrochlorid/Carbamoylchlorid (aus Amin und Phosgen in der Kälte gebildet) vorliegendem Amin und (iii) Phosgen bis zur Beendigung der zwischen Carbamoylchloridgruppen und Hydroxylgruppen ablaufenden Urethanbildungsreaktion bei Temperaturen unter 100°C, vorzugsweise unter 60°C zu halten. Im übrigen kann die

Carbonatbildung auch durch die Vermeidung eines zu hohen Phosgenüberschusses in der Anfangsphase der Phosgenierungsreaktion zurückgedrängt werden. Derartige Vorsichtsmaßnahmen erübrigen sich jedoch dann weitgehend, wenn eine gezielte Verlängerung der eingesetzten Alkohole über Carbonatbrücken erwünscht ist. Im allgemeinen wird eine derartige Verlängerung der Hydroxylkomponente jedoch nicht angestrebt.

Die Bildung von Harnstoffen beim erfindungsgemäßen Phosgenierungsverfahren kann im allgemeinen dadurch vermieden werden, daß man die gleichzeitige Anwesenheit von freien Aminogruppen und Carbamoylchloridgruppen ausschließt. Dies kann beispielsweise bei der Verwendung von freien Aminen als Ausgangsmaterialien dadurch erreicht werden, daß das Ausgangsamin sofort mit einer solchen Menge an Phosgen zusammengebracht wird, daß auf jedes Mol an primären Aminogruppen mindestens 0,5 Mol Phosgen entfallen.

Zur Durchführung des erfindungsgemäßen Verfahrens kann beispielsweise so vorgegangen werden, daß ein geeignetes Lösungsmittel vorgelegt und Phosgen bei ca. -20°C bis +10°C einkondensiert wird. Auf diese Weise werden beispielsweise 10 bis 200, vorzugsweise 35 bis 120 Gew.-Teile Phosgen pro 100 Gew.-Teilen Lösungsmittel enthaltende Lösungen hergestellt. Die so oder auf andere Art erhaltene Lösung wird dann bei ca. -20 bis 40°C, vorzugsweise 0 bis 25°C mit dem gegebenenfalls als Carbamat oder phosgenierbarem Salz (Hydrochlorid) vorliegenden Amin und der alkoholischen Komponente vereinigt.

0120254

Das so erhaltene Reaktionsgemisch wird dann vorteilhafterweise bei ca. 0 bis 40°C gehalten, bis die bei dieser Temperatur ablaufenden Reaktionen abgeschlossen sind. Anschließend wird das Reaktionsgemisch, vorzugsweise in Gegenwart eines Phosgenüberschusses (mehr als 1 Mol Phosgen pro Mol im Ausgangsamin vorliegender primärer Aminogruppen) zur Beendigung der Phosgenierungsreaktion auf 50-200°C, vorzugsweise 80 bis 180°C erhitzt. Der erforderliche Phosgenüberschuß kann beispielsweise durch fortlaufendes Einleiten von Phosgen sichergestellt werden.

Bei Verwendung von phosgenierbaren Salzen der zu phosgenierenden Amine (d.h. den entsprechenden Hydrochloriden) bzw. von Kohlendioxid-Addukten der zu phosgenierenden Amine (Carbamatmethode) können diese Ausgangsmaterialien und auch die alkoholische Komponente bereits während des Einkondensierens des Phosgen in das Lösungsmittel in diesem in Form einer Lösung bzw. Suspension vorliegen.

Gemäß einer weiteren Variante der Durchführung des erfindungsgemäßen Verfahrens erfolgt eine Überführung des zu phosgenierenden Amins in die entsprechenden Hydrochloride-Carbamoylchloride in Abwesenheit der alkoholischen Komponente bei -20 bis +40°C, bevorzugt bei 0 - 25°C, welche erst nach Beendigung dieser Kaltphosgenierung dem Reaktionsgemisch hinzugefügt wird.

Nach Beendigung der Phosgenierungsreaktion (Beendigung der Chlorwasserstoffabspaltung) wird das im Reaktionsgemisch befindliche Phosgen entfernt. Dies geschieht bevorzugt durch Einblasen eines inerten Gases wie trockenen Stickstoffs in das heiße oder gegebenenfalls z.B. 50 bis 100°C warme Reaktionsgemisch und/oder Anlegen von Vakuum, z.B. von 20 mbar. Vor oder bevorzugt nach der im wesentlichen Entfernung des Phosgens wird filtriert oder abgesaugt.

In Abhängigkeit vom Mengenverhältnis Amin/Alkohol entstehen auf diese Weise Lösungen von NCO-Prepolymeren bzw. NCO-Semiprepolymeren im zur Phosgenierung eingesetzten Lösungsmittel. Bei Verwendung eines Äquivalentverhältnisses zwischen Aminogruppen des zu phosgenierenden Amins und Hydroxylgruppen der Alkoholkomponente von unter 1:0,5 können auf diese Weise ohne Dünnschichtdestillation weitgehend monomerenfreie NCO-Prepolymere erhalten werden. Die Verfahrensprodukte können in Form ihrer Lösung im zur Phosgenierung eingesetzten Lösungsmittel oder nach dessen destillativer Entfernung in Substanz der üblichen Weiterverarbeitung zugeführt werden.

Bei den beschriebenen erfindungsgemäßen Verfahrensprodukten handelt es sich um Urethangruppen aufweisende Polyisocyanate, die im allgemeinen einen Gehalt an Carbonatgruppen -O-CO-O- von weniger als 2,0 Gew.-% und einen Gehalt an Harnstoffgruppen -NH-CO-NH- von weniger als 1,0 Gew.-% aufweisen. Der Gehalt der Verfahrensprodukte

an Isocyanatgruppen -NCO und Urethangruppen -O-CO-NH- kann in Abhängigkeit von Art und Mengenverhältnis der eingesetzten Ausgangsmaterialien innerhalb weiter Grenzen schwanken. Im allgemeinen liegt der NCO-Gehalt bei 0,5 bis 35, vorzugsweise 1 bis 25 Gew.-% und der Urethangehalt bei 0,5 bis 20, vorzugsweise 1 bis 4,0 Gew.-%.

Eine wesentliche Verbesserung der Herstellung von destillierbaren Polyisocyanaten wird durch die stark unterschüssige Mitverwendung von ein- und/oder mehrwertigen Alkoholen ermöglicht. Bei der Durchführung des erfindungsgemäßen Verfahrens unter Verwendung derartiger Alkohole werden im allgemeinen organische Diamine wie Hexamethylendiamin oder Diaminotoluole als Aminkomponente eingesetzt, so daß destillierbare Diisocyanate entstehen. Die ein- und/oder mehrwertigen Alkohole kommen hierbei vorzugsweise in einer solche Menge zum Einsatz, die einem Äquivalentverhältnis von primären Aminogruppen zu Hydroxylgruppen von 1:0,005 bis 1:0,2 entsprechen. Besonders bevorzugt wird eine solche Menge an Alkohol(en) verwendet, die genügt, um den ansonsten festen oder harzigen Destillationsrückstand zu verflüssigen. Das optimale Verhältnis und auch die Art des vorzugsweise einzusetzenden Alkohols wird der Fachmann durch einige wenige einfache Versuche ermitteln können. Es können sowohl einzelne Alkohole als auch beliebige Gemische von Alkoholen der oben unter a) - d) beispielhaft genannten Art eingesetzt werden.

Bei der destillativen Aufarbeitung der so erhaltenen Phosgenierungsprodukte verbleiben die Urethangruppen

aufweisenden, aus den Alkoholen entstandenen Derivate im Destillationsrückstand und verbessern dessen Fließverhalten, so daß diese Destillationsrückstände einfacher aus den Destillationsapparaturen ausgeschleust werden können.

Wie bereits dargelegt, können die nach dem erfindungsgemäßen Verfahren unter der bevorzugten Verwendung von mindestens zweiwertigen Alkoholen erhältlichen NCO-Prepolymeren oder NCO-Semiprepolymeren der üblichen Verwendung derartiger Produkte, d.h. insbesondere der Herstellung von Polyurethankunststoffen zugeführt werden. Bei dieser erfindungsgemäßen Verwendung der erfindungsgemäßen Verfahrensprodukte kann nach den an sich bekannten Methoden des einschlägigen Standes der Technik verfahren werden.

Die nach dem erfindungsgemäßen Verfahren erhältlichen Produkte können jedoch auch zur Herstellung der ihnen entsprechenden Polyamine (insbesondere Polyamino-polyether und/oder -polyester) verwendet werden. Bei dieser erfindungsgemäßen Verwendung können die erfindungsgemäßen Produkte beispielsweise entsprechend der Lehre der deutschen Offenlegungsschriften 2 948 419, 3 039 600, 3 129 979, 3 131 252, 3 223 395, 3 223 400, 3 223 398, 3 223 397, 3 227 219 oder 3 244 912 anstelle der in diesen Literaturstellen beschriebenen Ausgangsmaterialien durch hydrolytische Überführung der NCO-Gruppen in die entsprechenden Amine überführt werden.

In den nachfolgenden Beispielen beziehen sich alle Prozentangaben auf Gewichtsprozente.

Beispiel 1 (Herstellung eines Semiprepolymers)

In vorgelegten 1,5 l o-Dichlorbenzol werden bei ca. 0°C durch Einleiten 600 g gasförmiges Phosgen (6,06 mol) gelöst. Bei einer Temperatur im Bereich von 0-8°C wird eine 60°C warme Lösung von 150 g 4,4'-Diaminodiphenylmethan (0,76 mol) und 28,8 g Tripropylenglykol (0,15 mol) in 750 ml o-Dichlorbenzol zugegeben. Anschließend wird langsam auf 130°C erwärmt und gleichzeitig ein schwacher Phosgenstrom in das Reaktionsgefäß eingeleitet. Ab 80°C beginnt unter starker Chlorwasserstoff-Entwicklung Klärung der Lösung einzutreten. Nach 105 min. ist die Reaktion beendet und die Reaktionsphase fast völlig klar. Bei einer Innentemperatur von ca. 100°C wird mit einem Stickstoffstrom überschüssiges Phosgen ausgetrieben. Bei 20 mbar wird das o-Dichlorbenzol abdestilliert und der Rückstand heiß von wenigen Flocken ungelösten Materials abfiltriert. Letzte Spuren flüchtigen Materials wurden bei 0,1 mbar/100°C entfernt. Die Ausbeute ist quantitativ. Weitere Daten vgl. Tabelle 1.

Beispiel 2

In 700 ml Chlorbenzol werden bei ca. 0°C durch Einleiten 400 g gasförmiges Phosgen (4,04 mol) gelöst. Innerhalb 20 min. wird zu der mit Eis/Kochsalz-Kältemischung gekühlten Lösung eine Lösung aus 61 g 2,4-Diaminotoluol (0,5 mol) und 500 g eines Polypropylenglykols der OH-Zahl 56 (0,25 mol) in 400 ml Chlorbenzol zugegeben. Dann

wird für 150 min. auf Rückflußtemperatur erhitzt und gleichzeitig ein schwacher Phosgenstrom in das Reaktionsgefäß eingeleitet. Bei 50°C beginnt starke Gasentwicklung unter Aufklären der Reaktionsmischung. Durch Anlegen von Wasserstrahlpumpenvakuum wird das Chlorbenzol abdestilliert, von geringen, ungelösten Resten abfiltriert und bei 100°C/ 0,1 mbar nochmals flüchtige Bestandteile entfernt. Die Ausbeute beträgt 99 %.

Weitere Daten vgl. Tabelle 1.

Beispiel 3

In 500 ml Chlorbenzol werden bei ca. 0°C durch Einleiten 400 g gasförmiges Phosgen (4,04 mol) gelöst. Innerhalb 15 min. wird bei einem Innentemperaturbereich von 0-12°C eine 50°C warme Suspension von 54 g p-Phenylendiamin (0,5 mol) in einer Mischung aus 250 g eines Polypropylenglykols der OH-Zahl 112 und 500 ml Chlorbenzol zugegeben. Innerhalb 30 min. wird das Reaktionsgemisch auf Rückflußtemperatur (126°C) erhitzt und 150 min. bei dieser Temperatur gehalten. Beim Erreichen einer Innentemperatur von 50°C beginnt starke Chlorwasserstoff-Entwicklung. Währenddessen wird ständig ein schwacher Phosgenstrom in das Reaktionsgefäß eingeleitet. Nach Beendigung der Gasentwicklung wird durch Anlegen von 20 mbar Vakuum der größte Teil des Chlorbenzols abdestilliert. Geringfügige Reste unlöslicher Begleitprodukte werden abgesaugt und das NCO-Prepolymer nochmals bei 100°C/0,1 mbar von flüchtigen Stoffen befreit. Die Ausbeute ist quantitativ.

Weitere Daten vgl. Tabelle 1.

Beispiel 4

34,8 g (0,3 mol) Hexamethylendiamin werden in 300 ml o-Dichlorbenzol gelöst. Bei 90-95°C wird diese Lösung durch Einleiten von Kohlendioxid $CO_2$- gesättigt. Zur entstandenen Carbamat-Suspension werden 480 g eines trifunktionellen Polyethers der OH-Zahl 35, entstanden durch blockweise Addition von Propylenoxid und dann Ethylenoxid an Trimethylolpropan mit mindestens 95 % primären OH-Gruppen, zugefügt und die so entstandene Mischung auf 0°C heruntergekühlt. Bei dieser Temperatur werden 70 g Phosgen einkondensiert, dabei entweicht Kohlendioxid. Nach Beendigung der Kohlendioxidentwicklung wird das Reaktionsgemisch auf 160°C erhitzt und gleichzeitig ein schwacher Phosgenstrom in das Reaktionsgefäß eingeleitet. Nach einer Reaktionsdauer von 8 Stunden ist die Lösung klar. Bei einer von 160 auf 120°C fallenden Innentemperatur wird mit einem Stickstoffstrom 30 min. überschüssiges Phosgen ausgeblasen.

Bei 130°C/20 mbar und dann 120°C/0,1 mbar wird das Solvens abdestilliert und dann Spuren unlöslichen Produkts abgesaugt. Die Ausbeute ist quantitativ.

Weitere Daten vgl. Tabelle 1.

Tabelle 1

| | Bsp. 1 | Bsp. 2 | Bsp. 3 | Bsp. 4 |
|---|---|---|---|---|
| $NH_2$:OH-Äquivalent-Verhältnis | 5,05 | 2 | 2 | 2 |
| NCO-Wert (%) 1) | 23,6 | 3,7 | 6,8 | 2,4 |
| NCO-Wert (%) 2) | 23,3 | 3,6 | 6,62 | 2,4 |
| Carbonat 3) | - | - | Spuren | Spuren |
| Hydrolysierbares Cl (%) 4) | 0,17 | 0,05 | 0,05 | 0,11 |
| Viskosität (mPas) 5) | 750 | 2100 | 6050 | 12100 |
| Monomeren-Gehalt (%) 6) | 70 | 1,3 | 2,1 | 0,6 |

1) gefunden

2) berechnet

3) lt. IR als Bande bei 1780 $cm^{-1}$

4) nach Aufkochen mit methanolischer KOH Bestimmung nach Mohr

5) rotationsviskosimetrisch bei 24°C

6) gaschromatographisch

Beispiel 5

In 700 ml Chlorbenzol werden bei 0 - -10°C durch Einleiten 400 g gasförmiges Phosgen kondensiert. Innerhalb 30 min wird bei einer Innentemperatur von 5 - 18°C eine Lösung von 61 g (0,5 Mol) 2,4-Diaminotoluol, 100 g des Polypropylenglykol aus Beispiel 2 (0,05 Mol) und 400 ml Chlorbenzol zugetropft. Anschließend wird innerhalb 2 h auf 125°C erhitzt und gleichzeitig ein schwacher Phosgenstrom in das Reaktionsgefäß eingeleitet. Nach Beendigung der ab 50°C eingetretenen Chlorwasserstoff-Entwicklung wird überschüssiges Phosgen mit einem Stickstoffstrom ausgetrieben. Das Chlorbenzol wird im Vakuum abdestilliert. Aus dem Rückstand wird durch Anlegen von Ölpumpenvakuum 60 g reines 2,4-Toluylendiisocyanat abdestilliert (92-95°C/0,2 mbar), bei 0,2 mbar Druck und 200°C Badtemperatur geht kein weiteres 2,4-Diisocyantotoluol mehr über. Der Rückstand besteht aus 99 g einer Flüssigkeit mit einem NCO-Wert von 4,3 %, einer Viskosität bei 23°C von 3500 mPa.s und einem gaschromatographisch bestimmten Gehalt an freiem Toluylendiisocyanat von 1,4 %.

Beispiel 6

In 700 ml Chlorbenzol werden bei 0°C - -10°C 50 g (0,505 Mol) Phosgen kondensiert. In 20 min wird unter heftigem Rühren bei einer dabei von -5°C auf 19°C ansteigenden Innentemperatur eine 60°C heiße Lösung von 61 g (0,5 Mol) 2,4-Diaminotoluol in 500 ml Chlorbenzol zugetropft. Man rührt noch 15 min bei 20°C nach und erhält ein weißes

heterogenes Reaktionsgemisch. Anschließend werden bei dieser Temperatur unter Rühren 500 g (0,25 Mol) des Polypropylenglykols aus Beispiel 2 zugegeben und auf 120°C Innentemperatur hochgeheizt. Nach 90 min ist der Reaktionsansatz gelb gefärbt, weitere 3 h wird erhitzt. Währenddessen ist mäßige Gasabspaltung zu beobachten, restliches Gas wird durch einen Stickstoffstrom aus der Reaktionsmischung ausgeblasen. Der Fortgang der Reaktion kann im übrigen IR-spektroskopisch durch die Intensitätszunahme der Urethanbande bei 1722 $cm^{-1}$ und die Abnahme einer der 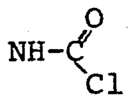 -Gruppe zugeordneten Bande bei 1775 $cm^{-1}$ beobachtet werden. Danach wird wieder abgekühlt und bei -5°C - 0°C weitere 350 g (3,54 Mol) Phosgen einkondensiert. Beim Aufheizen auf 125°C tritt ab 60°C Gasentwicklung ein. Gleichzeitig wird ein schwacher Phosgenstrom in das Reaktionsgefäß eingeleitet. Gegen Ende der Reaktion tritt Lösung ein. Überschüssiges Phosgen und Lösungsmittel werden wie im vorigen Beispiel entfernt. Es werden 551 g einer leicht trüben, braunen Flüssigkeit mit einer Viskosität von 6500 mPa.s/23°C und einem NCO-Wert von 3,8 % erhalten.

Beispiel 7 (Verwendungsbeispiel)

Vorgelegt wird eine Mischung aus 6,1 g (0,15 Mol) Natriumhydroxid, 50 ml Wasser und 60 ml Aceton. Unter intensivem Rühren werden dann in 10 min 99 g des NCO-Prepolymers aus Beispiel 5 mit einem NCO-Gehalt von 4,3 % (0,1 Mol) so zugegeben, daß die Innentemperatur 25°C nicht übersteigt. Dazu wird mit einem Eisbad gekühlt. Nach Beendigung der Zugabe wird 15 min bei 20°C nachgerührt und dann

1 h auf 80°C erhitzt. Beim Abkühlen scheiden sich zwei Phasen ab. Die untere Phase enthält die Hauptmenge an Salz und wird verworfen. Die obere Phase wird abgetrennt, Wasser und Aceton bei 100°C/20 - 1 mbar abdestilliert und der dunkelbraune Rückstand filtriert. Der Rückstand stellt ein oligomeres Polyamin mit einer Viskosität bei 25°C von 9900 mPa.s, einer NH-Zahl von 39,6 (mg KOH/g) und einem Gehalt an primärem Aminstickstoff von 0,99 % dar.

Patentansprüche

1. Verfahren zur Herstellung von Polyisocyanaten durch ein- oder zweistufige Phosgenierung von primären Polyaminen, gegebenenfalls nach ihrer an sich bekannten Überführung in phosgenierbare Salze oder Kohlendioxid-Addukte, dadurch gekennzeichnet, daß man die Phosgenierung zumindest in der zweiten Stufe in Gegenwart von mindestens einer mindestens eine primäre und/oder sekundäre alkoholische Hydroxylgruppe aufweisenden Verbindung unter Einhaltung eines Äquivalentverhältnisses von im Ausgangsamin vorliegenden, primären Aminogruppen zu Hydroxylgruppen von 1:0,005 bis 1:0,99 durchführt.

2. Verfahren gemäß Anspruch 1, dadurch gekennzeichnet, daß man die Phosgenierung zumindest in der 2. Stufe in Gegenwart von mindestens einer, mindestens eine primäre und/oder sekundäre alkoholische Hydroxylgruppe aufweisenden, Verbindung unter Einhaltung eines Äquivalentverhältnisses von im Ausgangsamin vorliegenden, primären Aminogruppen zu Hydroxylgruppen von 1:0,005 - 1:0,2 durchführt und nach der Phosgenierung überschüssiges monomeres Polyisocyanat abdestilliert.

3. Verfahren gemäß Anspruch 1 und 2, dadurch gekennzeichnet, daß man als primäre Polyamine diprimäre Diamine des Molekulargewichtsbereichs 60 bis 500 und als alkoholische Hydroxylgruppen aufweisende

Verbindung(en) mehrwertige Alkohole mit primären und/oder sekundären Hydroxylgruppen ausgewählt aus der Gruppe bestehend aus

a) Ether- und Estergruppen-freien Alkanpolyolen des Molekulargewichtsbereichs 62 bis 250,

b) Ether- und Estergruppen-freien Cycloalkanpolyolen des Molekulargewichtsbereichs 116 bis 250,

c) Ethergruppen aufweisenden Polyolen des Molekulargewichtsbereichs 106 bis 10 000,

d) Estergruppen aufweisenden Polyolen des Molekulargewichtsbereichs 206 bis 10 000 und

e) beliebigen Gemischen derartiger Verbindungen verwendet.

4. Verfahren gemäß Anspruch 1 bis 3, dadurch gekennzeichnet, daß man als Verbindung(en) mit alkoholisch gebundenen Hydroxylgruppen Polyole oder Polyolgemische einer (mittleren) Hydroxylfunktionalität von 2 bis 3 verwendet.

5. Verfahren gemäß Anspruch 1 bis 4, dadurch gekennzeichnet, daß man als primäre Polyamine diprimäre aromatische Diamine des Molekulargewichtsbereichs 108 bis 250 verwendet.

6. Gemäß Anspruch 1 bis 5 erhältliche Polyisocyanate.

7. Verwendung der gemäß Anspruch 1 bis 5 erhältlichen Polyisocyanate als Ausgangsmaterialien bei der Herstellung von Polyurethankunststoffen nach dem Isocyanat-Polyadditionsverfahren.

8. Verwendung der gemäß Anspruch 1 bis 5 erhältlichen Polyisocyanate zur Herstellung der ihnen entsprechenden Polyamine durch hydrolytische Überführung ihrer Isocyanatgruppen in Aminogruppen.

Europäisches Patentamt

# EUROPÄISCHER RECHERCHENBERICHT

**0120254**
Nummer der Anmeldung

EP 84 10 1435

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 014 723 (HOECHST)<br>* Seite 11, Ansprüche 1,2; Seite 1, Zeile 21 - Seite 2, Zeile 5; Seite 2, Zeile 31 - Seite 3, Zeile 25; Seite 8, Beispiel 9 *<br>--- | 1,3,5, 6 | C 08 G 71/04<br>C 08 G 18/72<br>C 08 G 18/83<br>C 07 C 125/06<br>C 07 C 118/02<br>C 08 G 18/30 |
| A | EP-A-0 050 275 (BAYER)<br>* Seite 33, Anspruch 1 *<br>----- | 8 | |
| | | | **RECHERCHIERTE SACHGEBIETE (Int. Cl. 3)** |
| | | | C 08 G<br>C 07 C |

Der vorliegende Recherchenbericht wurde für alle Patentansprüche erstellt.

| Recherchenort | Abschlußdatum der Recherche | Prüfer |
|---|---|---|
| DEN HAAG | 15-06-1984 | VAN PUYMBROECK M.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82